# EUROPÄISCHE PATENTSCHRIFT

(11) **EP 2 429 482 B1**
(45) Veröffentlichungstag und Bekanntmachung des Hinweises auf die Patenterteilung: **28.12.2016**
(21) Anmeldenummer: 10710574.4
(22) Anmeldetag: 22.03.2010
(51) Int. Cl.: A61K 8/44, A61K 8/46, A61K 8/73, A61K 8/86, A61Q 5/10

(54) **FÄRBEMITTEL MIT TENSID/EMULGATOR-KOMBINATION**
COLOURING AGENT WITH SURFACTANT/EMULSIFIER COMBINATION
AGENT DE COLORATION COMPRENANT UNE COMBINAISON TENSIOACTIF/ÉMULSIFIANT

(30) Priorität: 11.05.2009 DE 102009003002
(43) Veröffentlichungstag der Anmeldung: 21.03.2012
(73) Patentinhaber: Henkel AG & Co. KGaA, 40589 Düsseldorf (DE)
(72) Erfinder: REICHERT, Anja, 40629 Düsseldorf (DE); BENDER, Irmgard, 40595 Düsseldorf (DE); GRUNWALD, Mechtild, 40764 Langenfeld (DE); KLEEN, Astrid, 20457 Hamburg (DE)
(86) Internationale Anmeldenummer: PCT/EP2010/053659
(87) Internationale Veröffentlichungsnummer: WO 2010/130490

(56) Entgegenhaltungen:
- EP-A2- 2 143 419
- WO-A1-2006/136303
- WO-A2-2007/093271
- WO-A2-2008/022958
- WO-A2-2010/102928

## Beschreibung

Gegenstand der Erfindung ist ein Mittel zum oxidativen Färben von keratinischen Fasern, welches sich durch eine spezifische Kombination nichtionischer Emulgatoren mit unterschiedlichem Ethoxylierungsgrad und einer spezifischen Kombination grenzflächenaktiver Substanzen, die mindestens ein anionisches, mindestens ein zwitterionisches und mindestens ein amphoteres Tensid enthält. Die erfindungsgemäßen Mittel zeichnen sich durch gute Färbeeigenschaften bei gleichzeitigem Schutz vor Schädigungen für das menschliche Haar aus. Insbesondere lassen sich mit den erfindungsgemäßen Mitteln Färbungen mit hoher Farbintensität und eine verbesserte Grauabdeckung erzielen. Schließlich besitzen die erfindungsgemäßen Mittel signifikante Vorteile hinsichtlich der Viskositätsstabilität bei Lagerung und Anwendung sowie eine Verbesserung der Scherempfindlichkeit.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt. Als Kupplerkomponenten werden in der Regel m-Phenylendiaminderivate, Naphthole, Pyridinderivate, Resorcin und Resorcinderivate, Pyrazolone und m-Aminophenole verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. Bei insbesondere mehrfacher Anwendung von Mitteln mit 5,6-Dihydroxyindolin ist es möglich, Menschen mit ergrauten Haaren die natürliche Haarfarbe wiederzugeben.

Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Insbesondere oxidative Haarfärbemittel sind trotz ihrer vorteilhaften Färbeeigenschaften für den Anwender mit Nachteilen behaftet.

Erstens führen der Einsatz der Oxidationsmittel zur Entwicklung der eigentlichen Färbung und der zur Ausfärbung notwendige basische pH-Wert der Mittel zu Schädigungen in der Haarstruktur und auf der Haaroberfläche. Andererseits besteht weiterhin Bedarf nach Mitteln mit verbesserten Färbeleistungen. Insbesondere Mittel für verbesserte Färbungen mit hoher Intensität und Farbigkeit sowie gutem Grauabdeckungsvermögen, insbesondere auf unterschiedlich vorbehandelten Haaren, sind weiterhin im Focus der Entwicklung.

Schließlich hat sich gezeigt, dass gängige Haarfärbemittel aufgrund der Vielzahl der verwendeten Inhaltsstoffe häufig unbefriedigende rheologische Eigenschaften besitzen. Diese drücken sich insbesondere in Viskositätsschwankungen während der Lagerung und der Anwendung aus. So sind Mittel, die im Verlauf der Lagerung ausdicken, mit Problemen bei Entnahme, Dosierung und Anwendung verbunden, während Mittel, die eine Viskositätserniedrigung während der Lagerung erfahren, ebenfalls ungenügende Anwendungseigenschaften besitzen. Schließlich ist es vorteilhaft, Mittel mit verbesserter Scherempfindlichkeit bereitzustellen.

Aufgabe der vorliegenden Erfindung ist es daher, die oben genannten Nachteile oxidativer Haarfärbemittel herabzusenken. Die Färbemittel sollen eine verringerte Schädigung des Haares bewirken. Insbesondere Schutz vor oxidativen Schädigungen der Haarstruktur und der Haaroberfläche soll durch die Haarfärbemittel erzielt werden. Die Färbemittel sollen gleichzeitig über verbesserte rheologische Eigenschaften verfügen, die sich insbesondere in verbesserter Viskositätsstabilität und Scherempfindlichkeit ausdrücken. Weiterhin soll das Färbemittel eine verbesserte Farbintensität und eine verbesserte Grauabdeckung gegenüber herkömmlichen Färbemitteln, bevorzugt bei einer Reduktion des pH-Werts, ermöglichen.

In nicht vorhersehbarer Weise wurde nun gefunden, dass eine neuartige Färbecremegrundlage auf Basis einer Kombination von hochethoxylierten Fettalkohol-Emulgatoren mit unterschiedlich hohen Ethoxylierungsgraden und einer Kombination aus grenzflächenaktiven Verbindungen, enthaltend mindestens ein anionisches Tensid, mindestens ein zwitterionisches Tensid und mindestens ein amphoteres Tensid, die Bereitstellung von Färbemitteln mit hoher Viskositätsstabilität ermöglicht.

Erster Gegenstand der vorliegenden Erfindung ist daher ein zum Färben keratinischer Fasern, insbesondere menschlicher Haare, enthaltend in einem kosmetischen Träger mindestens ein Oxidationsfarbstoffvorprodukt, sowie
a) mindestens eine nichtionischen Emulgatorkombination aus mindestens zwei ethoxylierten, linearen Fettalkoholen mit jeweils 8 bis 22 Kohlenstoffatomen, wobei mindestens einer der ethoxylierten Fettalkohole einen mittleren, durchschnittlichen Ethoxylierungsgrad von 15 bis 35 Mol Ethylenoxid besitzt und mindestens einer der ethoxylierten Fettalkohole einen hohen, durchschnittlichen Ethoxylierungsgrad von 40 bis 100 Mol Ethylenoxid besitzt, und
b) mindestens eine Kombination grenzflächenaktiver Substanzen, enthaltend mindestens ein anionisches Tensid, mindestens ein zwitterionisches Tensid und mindestens ein amphoteres Tensid,
und dadurch gekennzeichnet, dass die Kombination grenzflächenaktiver Substanzen ein Gewichtsverhältnis zwischen der Gesamtmenge an anionischen Tensiden und der Gesamtmenge an amphoteren und zwitterionischen Tensiden besitzt, dessen Wert zwischen 3 und 0,5 liegt.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten.

Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten wesentlichen Inhaltsstoff enthält das erfindungsgemäße Mittel mindestens eine Emulgatorkombination aus mindestens zwei ethoxylierten, linearen Fettalkoholen jeweils mit 8 bis 22 Kohlenstoffatomen, wobei mindestens einer der ethoxylierten Fettalkohole einen mittleren, durchschnittlichen Ethoxylierungsgrad von 15 bis 35 Mol Ethylenoxid besitzt und mindestens einer der ethoxylierten Fettalkohole einen hohen, durchschnittlichen Ethoxylierungsgrad von 40 bis 100 Mol Ethylenoxid besitzt

Die nichtionischen Emulgatoren sind dabei ausgewählt aus ethoxylierten, linearen Fettalkoholen, bevorzugt einer Kettenlänge mit 8 bis 22 Kohlenstoffatomen. Unter einem ethoxylierten Fettalkohol wird im Sinne der Erfindung ein Anlagerungsprodukt von Ethylenoxid an einen Fettalkohol verstanden, wobei der Ethoxylierungsgrad die Molmenge Ethylenoxid (EO) angibt, die durchschnittlich pro Mol Fettalkohol angelagert wurde. Es ist überraschend gefunden worden, dass die Kombination aus mindestens einem ethoxylierten Fettalkohol mit einen mittleren, durchschnittlichen Ethoxylierungsgrad und mindestens einem ethoxylierten Fettalkohol mit einen hohen, durchschnittlichen Ethoxylierungsgrad mit einer Verbesserung der Viskositätsstabilität einhergeht und eine hervorragende Lagerstabilität der Mittel gewährleistet.

Bevorzugte ethoxylierte Fettalkohole sind Ethylenoxid-Anlagerungsprodukte an Caprinalkohol, Laurylalkohol, Isotridecylalkohol, Myristylalkohol, Cetylalkohol, Palmoleylalkohol, Stearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Besonders bevorzugt sind Anlagerungsprodukte an technische Fettalkohole bzw. deren Mischungen mit 12 bis 18 Kohlenstoffatomen wie beispielsweise Kokos-, Palm-, Palmkern- oder Talgfettalkohol, insbesondere Kokos- und/ oder Talgfettalkohol.

Unter ethoxylierten Fettalkohol mit einem mittleren, durchschnittlichen Ethoxylierungsgrad wird im Sinne der Erfindung ein Anlagerungsprodukt von 15 bis 35 Mol Ethylenoxid pro Mol Fettalkohol verstanden. Ein ethoxylierter Fettalkohol mit einem hohen, durchschnittlichen Ethoxylierungsgrad ist im Sinne der Erfindung ein Anlagerungsprodukt von 40 bis 100 Mol Ethylenoxid pro Mol Fettalkohol verstanden. Bevorzugte Fettalkohole mit einem hohen, durchschnittlichen Ethoxylierungsgrad besitzen einen Ethoxylierungsgrad von 40 bis 60, besonders bevorzugt von 45 bis 55.

Je nach Herstellungsmethode fallen die erfindungsgemäßen ethoxylierten Fettalkohole als ein Gemisch mit einer unterschiedlichen Ethoxylierungsgradverteilung an. Im Sinne der Erfindung werden diese Emulgatoren daher nach dem durchschnittlichen Ethoxylierungsgrad gekennzeichnet. Dieser ist üblicherweise als Ziffer hinter dem Fettalkohol-Suffix "eth-" in der INCl-Bezeichnung erkennbar.

Bevorzugte ethoxylierte Fettalkohole mit einem mittleren, durchschnittlichen Ethoxylierungsgrad sind beispielsweise die Handelsprodukte Ceteth-15 (Nikkol BC 15 TX, Firma Nikko Chemicals Co., Ltd.), Laneth-15 (Polychol 15, Firma Croda), Ceteareth-15 (Eumulgin CS 15, Firma Cognis); Laneth-16 (und) Ceteth-16 (und) Oleth-16 (und) Steareth-16 (als Gemisch vertrieben unter dem Handelsnamen Solulan 16, Firma Noveon); Oleth-20 (Ritoleth 20, Firma Rita Corp.), Ceteth-20 (Brij 58 SP, Firma Uniqema; Lipocol C 20, Firma Lipo Chemicals Inc.), Ceteareth-20 (Surfac JH 200, Firma Surfachem; Eumulgin B 2, Firma Cognis), Laneth-20 (Polychol 20, Firma Croda); Steareth-21 (Brij 721 P, Firma Uniqema; Eumulgin S 21, Firma Cognis); Ceteareth-23 (Mergital C 23, Firma Cognis), Laureth-23 (Canasol BJ 35, Firma Canamax); Ceteareth-25 (Cremophor A 25, Firma BASF); Ceteareth-27 (Plurafac A 38, Firma BASF); Ceteareth-30 (Lipocol SC 30, Firma Lipo Chemicals; Eumulgin B 3, Firma Cognis).

Bevorzugte ethoxylierte Fettalkohole mit einem hohen, durchschnittlichen Ethoxylierungsgrad sind beispielsweise die Handelsprodukte: Ceteth-40 (Nikkol BC 40 TX, Firma Nikko Chemicals Co., Ltd.), Laneth-40 (Polychol 40, Firma Croda); Oeth-50 (Nikkol BO 50 V, Firma Nikko Chemicals Co., Ltd.), Ceteareth-50 (Genapol T 500, Firma Clariant; Mergital CS 50, Firma Cognis); Ceteareth-60 (Findet 1618 A 72, Firma KAO Corp.); Ceteareth-80 (Lutensol AT 80, Firma BASF). Besonders bevorzugt ist Ceteareth-50.

Ethoxylierte Fettalkohole mit mittlerem und hohem, durchschnittlichen Ethoxylierungsgrad sind im anwendungsbereiten Mittel insgesamt zwischen 0,05 und 15 Gew.-%, bevorzugt 0,1 und 10 Gew.-% enthalten.

Erfindungsgemäß besonders bevorzugte Mittel sind dadurch gekennzeichnet, dass das Gewichtsverhältnis der Menge der ethoxylierten Fettalkohole mit einem mittleren, durchschnittlichen Ethoxylierungsgrad zu der Menge der ethoxylierten Fettalkohole mit einem hohen, durchschnittlichen Ethoxylierungsgrad im Färbemittel (a) einen Wert von 3:1 bis 1:5, bevorzugt von 2:1 bis 1:3, besitzt.

Als weiteren wesentlichen Inhaltsstoff enthalten die erfindungsgemäßen Mittel mindestens eine Kombination aus grenzflächenaktiven Substanzen, die mindestens ein anionisches Tensid, mindestens ein zwitterionisches Tensid und mindestens ein amphoteres Tensid umfasst.

Geeigneten anionische oberflächenaktiven Stoffe sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel RO(CH₂CH₂O)ₓCH₂COOH, in der R eine lineare Alkylgruppe mit 8 bis 30 C-Atomen und x = 0 oder einer Zahl von 1 bis 16 ist,
- Acylsarcoside, Acyltauride und/oder Acylisethionate jeweils mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate, lineare α-Olefinsulfonate, und Sulfonate ungesättigter Fettsäuren mit jeweils 8 bis 24 C-Atomen und eventuell 1 bis 6 Doppelbindungen,
- α-Sulfofettsäuremethylester von Fettsäuren mit 8 bis 30 C-Atomen,
- Alkylsulfate und Alkylethersulfate der Formel RO(CH₂CH₂O)ₓSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen,
- Alkyl- und/oder Alkenyletherphosphate der Formel 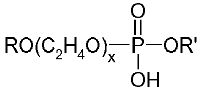 in der R bevorzugt für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht,
- sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht,
- Monoglyceridsulfate und Monoglyceridethersulfate.

Insbesondere enthalten die erfindungsgemäßen Färbemittel anionische Tenside, ausgewählt aus Fettsäuren, Alkylsulfaten, Alkylethersulfaten und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Erfindungsgemäß bevorzugte amphotere Tenside sind Verbindungen gemäß Formel (I) und/oder dessen physiologisch verträglichem Salze, 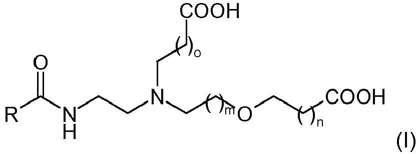 worin R für eine lineare oder verzweigte, gesättigte oder ungesättigte C₁₀-C₂₂-Alkylgruppe steht und m, n und o jeweils unabhängig voneinander für eine ganze Zahl 1, 2 oder 3 steht. In einer bevorzugten Ausführungsform steht der Rest R gemäß Formel (I) für eine lineare C₁₂-C₁₈-Alkylgruppe. Besonders bevorzugte Verbindungen der Formel (I) werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) vermarktet. Dabei leitet sich die Gruppe R-CO- gemäß Formel (I) von Fettsäuren des Kokosnussöls ab.

Eine Ausführungsform der vorliegenden Erfindung zeichnet sich dadurch aus, dass die Kombination grenzflächenaktiver Substanzen aus
mindestens Alkylethersulfat als anionischem Tensid;
mindestens Cocamidopropyl Betaine als zwitterionischem Tensid
und mindestens Disodium Cocoamphodipropionate als amphoterem Tensid besteht.

Eine weitere Ausführungsform der vorliegenden Erfindung zeichnet sich dadurch aus, dass die Kombination grenzflächenaktiver Substanzen aus
mindestens Alkylsulfat als anionischem Tensid;
mindestens Cocamidopropyl Betaine als zwitterionischem Tensid
und mindestens Disodium Cocoamphodipropionate als amphoterem Tensid besteht.

Eine weitere Ausführungsform der vorliegenden Erfindung zeichnet sich dadurch aus, dass die Kombination grenzflächenaktiver Substanzen aus
mindestens einer Ethercarbonsäure als anionischem Tensid;
mindestens Cocamidopropyl Betaine als zwitterionischem Tensid
und mindestens Disodium Cocoamphodipropionate als amphoterem Tensid besteht.

Eine weitere Ausführungsform der vorliegenden Erfindung zeichnet sich schließlich dadurch aus, dass die Kombination grenzflächenaktiver Substanzen aus
mindestens einer Fettsäure als anionischem Tensid;
mindestens Cocamidopropyl Betaine als zwitterionischem Tensid
und mindestens Disodium Cocoamphodipropionate als amphoterem Tensid besteht.

Die anionischen, amphoteren und zwitterionischen Tenside sind in dem erfindungsgemäßen Mittel bevorzugt in einer Gesamtmenge von 0,05 bis 30 Gew.-%, besonders bevorzugt von 0,1 bis 20 Gew.-%, jeweils bezogen auf das Gesamtgewicht des Mittels, enthalten.

Eine Ausführungsform der vorliegenden Erfindung ist dadurch gekennzeichnet, dass die Kombination grenzflächenaktiver Substanzen ein Gewichtsverhältnis zwischen der Gesamtmenge an anionischen Tensiden und der Gesamtmenge an amphoteren und zwitterionischen Tensiden besitzt, dessen Wert zwischen 2 und 0,5 liegt jeweils bezogen auf das Gesamtgewicht des anwendungsbereiten Mittels.

Schließlich enthalten die erfindungsgemäßen Mittel mindestens ein Oxidationsfarbstoffvorprodukt. Die erfindungsgemäßen Mittel zum Färben keratinischer Fasern sind demnach dadurch gekennzeichnet, dass sie mindestens ein Oxidationsfarbstoffvorprodukt vom Entwicklertyp und/oder Kupplertyp enthalten. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen. Dabei werden Entwicklerkomponenten und Kupplerkomponenten im Allgemeinen in etwa molaren Mengen zueinander eingesetzt.

Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Geeignete erfindungsgemäße Entwicklerkomponenten sind p-Phenylendiaminderivate, insbesondere ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxy-ethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylen-diamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylen-diamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß bevorzugte Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)ethylendiamin, N,N'-Bis-(4'-aminophenyl)-tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethyl-phenol, 4-Amino-2-(2-hydroxyethoxy)-phenol, 4-Amino-2-methylphenol, 4-Amino-2-hydroxy-methylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethyl-aminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)-phenol und 4-Amino-2-(diethylaminomethyl)phenol.

Ferner kann die Entwicklerkomponente ausgewählt sein aus o-Aminophenol und seinen Derivaten, wie 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten, Pyrazolopyrazol-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triamino-pyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)-pyrazol, 3,4-Diaminopyrazol, 4,5-Diamino-1-(4'-chlor-benzyl)-pyrazol, 4,5-Diamino-1,3-dimethylpyrazol, 4,5-Diamino-3-methyl-1-phenylpyrazol, 4,5-Di-amino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Di-amino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze. Unter den Pyrazolo[1,5-a]pyrimidinen sind insbesondere Pyrazolo[1,5-a]pyrimidin-3,7-diamin; 2,5-Dimethylpyrazolo[1,5-a]pyrimidin-3,7-diamin; Pyrazolo[1,5-a]pyrimidin-3,5-diamin; 2,7-Dimethyl-pyrazolo[1,5-a]pyrimidin-3,5-diamin; 3-Aminopyrazolo[1,5-a]pyrimidin-7-ol; 3-Aminopyrazolo[1,5-a]pyrimidin-5-ol; 2-(3-Aminopyrazolo[1,5-a]pyrimidin-7-ylamino)ethanol; 2-(7-Aminopyrazolo[1,5-a]pyrimidin-3-ylamino)ethanol; 2-[(3-Aminopyrazolo[1,5-a]pyrimidin-7-yl)-(2-hydroxyethyl)amino]ethanol; 2-[(7-Aminopyrazolo[1,5-a]pyrimidin-3-yl)-(2-hydroxyethyl)amino]ethanol; 5,6-Dimethylpyrazolo[1,5-a]pyrimidin-3,7-diamin; 2,6-Dimethylpyrazolo[1,5-a]pyrimidin-3,7-diamin; 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist, geeignet. Bevorzugte Pyrazolopyrazol-Derivate sind dabei 2,3-Diamino-6,7-dihydro-1H,5H-pyrazolo[1,2-a]pyrazol-1-on und 2,3-Diamino-6,7-dihydro-8,8-dimethyl-1H,5H-pyrazolo[1,2-a]pyrazol-1-on.

Besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxy-ethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on, sowie den physiologisch verträglichen Salzen dieser Verbindungen.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxylgruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten stellen m-Aminophenol und/oder dessen Derivate dar, bevorzugt ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methyl-phenol, 2-Hydroxy-4-aminophenoxyethanol, 2,6-Dimethyl-3-aminophenol, 3-Trifluoracetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol und den physiologisch verträglichen Salzen dieser Verbindungen.

Erfindungsgemäße Kupplerkomponenten stellen m-Diaminobenzol und/oder Derivate dar, insbesondere ausgewählt aus mindestens einer Verbindung aus der Gruppe, die gebildet wird aus m-Phenylendiamin, 2-(2,4-Diaminophenoxy)ethanol, 1,3-Bis-(2,4-diaminophenoxy)propan, 1-Methoxy-2-amino-4-(2'-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}-amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und den physiologisch verträglichen Salzen der genannten Verbindungen.

Erfindungsgemäße Kupplerkomponenten stellen o-Diaminobenzole dar, ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Kupplerkomponenten vom Typ der Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Erfindungsgemäße Kupplerkomponenten stellen weiterhin Pyridinderivate dar, vorzugsweise ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diamino-pyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Erfindungsgemäße Kupplerkomponenten stellen weiterhin Naphthalinderivate mit mindestens einer Hydroxygruppe, insbesondere ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxy-naphthalin.

Erfindungsgemäße Kupplerkomponenten stellen Indolderivate und Indolinderivate, bevorzugt ausgewählt aus mindestens einer Verbindung der Gruppe, die gebildet wird aus 4-Hydroxyindol, 6-Hydroxyindol, 7-Hydroxyindol, 4-Hydroxyindolin, 6-Hydroxyindolin und 7-Hydroxyindolin und den physiologisch verträglichen Salzen der vorgenannten Verbindungen.

Erfindungsgemäße Kupplerkomponenten stellen weiterhin Pyrimidinderivate dar, insbesondere ausgewählt aus mindestens einer Verbindung der Gruppe, die gebildet wird aus 4,6-Diamino-pyrimidin, 4-Amino-2,6-dihydroxypyrimidin, 2,4-Diamino-6-hydroxypyrimidin, 2,4,6-Trihydroxy-pyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäße Kupplerkomponenten stellen schließlich o-Aminophenolderivate, wie beispielsweise o-Aminophenol, Pyrazolonderivate, wie beispielsweise 1-Phenyl-3-methylpyrazol-5-on, Morpholinderivate, wie beispielsweise 6-Hydroxybenzomorpholin oder 6-Amino-benzomorpholin, Chinoxalinderivate wie beispielsweise 6-Methyl-1,2,3,4-tetrahydrochinoxalin und den physiologisch verträglichen Salzen der vorgenannten Verbindungen dar.

Erfindungsgemäß bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)amino-2-methylphenol, 2,4-Dichlor-3-amino-phenol, 2-Aminophenol, 3-Phenylendiamin, 2-(2,4-Diaminophenoxy)ethanol, 1,3-Bis(2,4-diamino-phenoxy)propan, 1-Methoxy-2-amino-4-(2-hydroxyethylamino)benzol, 1,3-Bis(2,4-diaminophenyl)-propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxy-indolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Färbemittel, verwendet.

Erfindungsgemäße geeignete Mittel können weiterhin zusätzlich mindestens einen polymeren Verdicker enthalten. Dadurch lässt sich sie Rheologie der erfindungsgemäßen Mittel lässt sich in die vom Verbraucher gewünschten cremig-fließfähigen Systeme überführen.

Bevorzugt handelt es sich bei dem polymeren Verdicker um einen hydrophilen Verdicker. Dem Fachmann sind solche Verdicker bekannt. Erfindungsgemäß einsetzbare, polymere Verdicker sind unter anderem
- synthetische Polymere, insbesondere Polyacrylsäurepolymere (Carbomere), insbesondere Poly(meth)acrylate, weiterhin gegebenenfalls verzweigte Homo- oder Co-Polymere der Acrylsäure, ihrer Salze oder ihrer Alkylester, die gegebenenfalls kationisch oder anionisch in der Alkylkette modifiziert sind; Polyacrylamidpolymere, insbesondere Homo- oder Co-Polymere von Acrylsäureamid und/oder Methacrylsäureamid, die gegebenenfalls kationisch oder anionisch modifiziert sind; und Copolymere aus Acrylsäure und Acrylsäureamid;
- polysaccharidische Polymere, wie beispielsweise Algin, Alginate, Glucane wie Dextran, Pullulan, Curdlan, Cellulose, Laminarin, Amylose oder Lichenin, Traganth, Karaya-Gummi, Ghatti-Gummi, Agar, Carrageenan, Chitin, Chitosan, Gummi arabicum, Gellan, Carob Gummi, Galactonmannane wie Guar oder Johannisbrotkernmehl, Tamarindenkernmehl oder deren Derivate;
- anorganische Verdicker, insbesondere geeignete Elektrolyte, wie Natriumchlorid oder Kaliumchlorid, Schichtsilicate (polymere, kristalline Natriumdisilicate) und Magnesium Aluminium Silicate oder Bentonite, besonders Smektite, wie Montmorillonit oder Hectorit, die gegebenenfalls auch geeignet modifiziert sein können;
sowie deren Mischungen.

Es hat sich gezeigt, dass insbesondere verdickende, polysaccharidische Polymere milde und lagerstabile Formulierungen ergeben. Erfindungsgemäß bevorzugte polymere Verdicker sind daher verdickende, polysaccharidische Polymere. Besonders bevorzugte Polysaccharide sind dabei Polymere auf Cellulose-Basis. Hierbei können chemisch modifizierten Cellulosen, wie beispielsweise Acetyl-, Methyl- oder Ethylcellulosen, Hydroxyalkylcellulosen oder Carboxyalkylcellulosen eingesetzt werden. Besonders bevorzugte Cellulosederivate besitzen saccharidische Seitenketten, wie beispielsweise Xanthan.

Eine Ausführungsform der vorliegenden Erfindung ist dadurch gekennzeichnet, dass der polymere Verdicker des Mittels ein polysaccharidisches Polymer, bevorzugt Xanthan, ist.

Erfindungsgemäß bevorzugte Mittel sind dadurch gekennzeichnet, dass der polymerer Verdicker zu 0,005 bis 1,0 Gew.-%, insbesondere zu 0,01 bis 0,5 Gew.-%, und ganz besonders zu 0,05 bis 0,3 Gew.-%, jeweils bezogen auf das Gesamtgewicht der Anwendungszubereitung, enthalten ist.

Um die Keratinfasern und auch die Haut so weit wie möglich zu schonen, ist die Einstellung eines zu hohen pH-Wertes nicht wünschenswert. Ein weiterer wesentlicher Aspekt der erfindungsgemäßen Mittel ist daher, dass das gebrauchsfertige Haarfärbepräparat einen pH-Wert im Bereich von 7 bis 11 aufweist. Besonders bevorzugt ist die Anwendung der Haarfärbemittel in einem schwach alkalischen Milieu, bevorzugt bei einem pH-Wert im Bereich von 7,5 bis 9,5. Bei den pH-Werten im Sinne der vorliegenden Erfindung handelt es sich um pH-Werte, die bei einer Temperatur von 25 °C gemessen wurden.

Zur Einstellung des pH-Werts sind dem Fachmann gängige Acidifizierungs- und Alkalisierungsmittel geläufig. Die zur Einstellung des bevorzugten pH-Wertes erfindungsgemäß verwendbaren Alkalisierungsmittel können aus der Gruppe, die gebildet wird aus basischen Aminosäuren, Aminen, Ammoniak, Alkalimetallhydroxiden, Alkalimetallmetasilikaten, Alkalimetallphosphaten und Alkalimetallhydrogenphosphaten ausgewählt werden. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Es hat sich herausgestellt, dass Mittel, die zwei unterschiedliche Alkalisierungsmittel enthalten, besonders geeignet sind, insbesondere, wenn eines davon eine basische Aminosäure ist.

Eine weitere Ausführungsform des erfindungsgemäßen Mittels ist dadurch gekennzeichnet, dass das Mittel eine Kombination von mindestens zwei Alkalisierungsmitteln enthält, wobei mindestens ein Alkalisierungsmittel ein Alkanolamin ist, ausgewählt aus der Gruppe, die gebildet wird aus Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol, 2-Aminobutanol, N,N-Dimethyl-ethanolamin, Methylglucamin, Triethanolamin, Diethanolamin und Triisopropanolamin, und wobei mindestens ein Alkalisierungsmittel eine basische Aminosäure ist.

Besonders bevorzugt sind dabei Mittel, die als Alkanolamin Monoethanolamin enthalten.

Das Alkanolamin ist in den erfindungsgemäßen Oxidationsfärbemitteln bevorzugt in Mengen von 0,5 bis 15 Gew.-%, insbesondere von 2 bis 5 Gew.-%, bezogen auf das Gesamtgewicht der Anwendungsmischung, enthalten.

Als basische Aminosäure im Sinne der Erfindung gilt eine organische Verbindung, die in ihrer Struktur mindestens eine protonierbare Aminogruppe und mindestens eine Carboxylat oder eine Sulfonat-Gruppe aufweist, und deren isoelektrischer Punkt (pl) einen Wert von 7,0 oder größer, gemessen unter Standardbedingungen, besitzt. Bevorzugte basische Aminosäuren sind Aminocarbonsäuren, insbesondere α-Aminocarbonsäuren (alpha-Aminocarbonsäuren) und ω-Amino-carbonsäuren. Unter den α-Aminocarbonsäuren sind wiederum Lysin, Ornithin, Guanidin und Arginin besonders bevorzugt.

Die basischen Aminosäuren können den erfindungsgemäßen Mitteln bevorzugt in freier Form zugegeben werden. In einer Reihe von Fällen ist es jedoch auch vorteilhaft, die Aminosäuren in Salzform einzusetzen. Bevorzugte Salze sind dann die Verbindungen mit Halogenwasserstoffsäuren oder Schwefelsäure, insbesondere die Hydrochloride, die Hydrobromide und die Sulfate. Weiterhin können die basischen Aminosäuren auch in Form von Oligopeptiden und Proteinhydrolysaten eingesetzt werden, wenn sichergestellt ist, dass die erforderlichen Mengen der erfindungsgemäß eingesetzten Aminosäuren darin enthalten sind.

Eine erfindungsgemäß besonders bevorzugte basische Aminosäure ist Arginin, insbesondere in freier Form, aber auch als Hydrochlorid eingesetzt.

Die basische Aminosäure ist in den erfindungsgemäßen Oxidationsfärbemitteln bevorzugt in Mengen von 0,01 bis 10 Gew.-%, insbesondere von 0,05 bis 5 Gew.-%, bezogen auf das Gesamtgewicht der Anwendungsmischung, enthalten.

Weiterhin können die erfindungsgemäßen Mittel als farbverändernde Komponente mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe sind als anionische, kationische und nichtionische direktziehende Farbstoffe bekannt. Eine weitere Ausführungsform der vorliegenden Erfindung ist daher dadurch gekennzeichnet, dass das Mittel zusätzlich mindestens einen direktziehenden Farbstoff enthält.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitro-phenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphtho-chinon, Pikraminsäure und deren Salze, 2-Amino-6-chloro-4-nitrophenol, 4-Ethylamino-3-nitrobenzoesäure und 2-Chlor-6-ethylamino-4-nitrophenol.

In einer weiteren Ausführungsform der vorliegenden Erfindung enthalten die Mittel zusätzlich mindestens eine Farbstoffvorstufe naturanaloger Farbstoffe.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%. Bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, insbesondere N-Methyl-5,6-dihydroxyindolin, N-Ethyl-5,6-dihydroxyindolin, N-Propyl-5,6-dihydroxyindolin, N-Butyl-5,6-dihydroxyindolin und besonders bevorzugt 5,6-Dihydroxyindolin. Bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, vorzugsweise N-Methyl-5,6-dihydroxyindol, N-Ethyl-5,6-dihydroxyindol, N-Propyl-5,6-dihydroxyindol, N-Butyl-5,6-dihydroxyindol sowie insbesondere 5,6-Dihydroxyindol.

Es ist nicht erforderlich, dass Oxidationsfarbstoffvorprodukte, direktziehende Farbstoffe oder naturanaloge Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder dessen Anlagerungsprodukte an Harnstoff, Melamin sowie Natriumborat in Frage. Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und anorganischen Persulfaten eingesetzt, woraus eine Steigerung des Aufhellvermögens der Mittel resultiert. Bevorzugte Persulfatsalz sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Der Einsatz von Persulfatsalzen bzw. Peroxodisulfatsalzen erfolgt in der Regel in Form eines gegebenenfalls entstaubten Pulvers oder eines in Form gepressten Formkörpers.

Bei einer Anwendung von zusätzlichen Oxidationsmitteln wird das eigentliche Färbemittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt, sowie einer Zubereitung, enthaltend das zusätzliche Oxidationsmittel, insbesondere Wasserstoffperoxid, hergestellt. Das erfindungsgemäße Mittel kann dabei die Kombination aus nichtionischer Emulgatorkombination aus mindestens zwei ethoxylierten, linearen Fettalkoholen und Kombination grenzflächenaktiver Substanzen, enthaltend mindestens ein anionisches Tensid, mindestens ein zwitterionisches Tensid und mindestens ein amphoteres Tensid, in der Zubereitung mit Oxidationsfarbstoffvorprodukten und/oder in der Oxidationsmittelzubereitung enthalten. Bevorzugt enthält die Zubereitung mit Oxidationsfarbstoffvorprodukten eine Kombination aus nichtionischer Emulgatorkombination aus mindestens zwei ethoxylierten, linearen Fettalkoholen und Kombination grenzflächenaktiver Substanzen, enthaltend mindestens ein anionisches Tensid, mindestens ein zwitterionisches Tensid und mindestens ein amphoteres Tensid, gemäß dem ersten Erfindungsgegenstand.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.-%, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Weiterhin hat es sich als vorteilhaft erweisen, wenn die Oxidationsmittelzubereitungen, insbesondere aus Oxidationsfärbemitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure.

Die erfindungsgemäßen Mittel enthalten weitere Hilfs- und Zusatzstoffe.

Weiterhin hat es sich als vorteilhaft erwiesen, wenn die erfindungsgemäßen Mittel neben den oben beschriebenen nichtionischen Emulgatoren zusätzliche nichtionogene grenzflächenaktive Stoffe enthalten. Solche Verbindungen sind beispielsweise C₁₂-C₃₀-Fettsäuremono- und -diester von Anlagerungsprodukten von 1 bis 30 Mol Ethylenoxid an Glycerin; Polyglycerinester und alkoxylierte Polyglycerinester, wie beispielsweise Poly(3)glycerindiisostearat und Poly(2)glycerinpolyhydroxy-stearat; alkoxylierte, bevorzugt propoxylierte und insbesondere ethoxylierte, Mono-, Di- und Triglyceride, wie beispielsweise Glycerinmonolaurat + 20 EO (Mol Ethylenoxid) und Glycerinmonostearat + 20 EO; Aminoxide; Sorbitanfettsäureester und Anlagerungeprodukte von Ethylenoxid an Sorbitanfettsäureester wie beispielsweise Polysorbate, Sorbitanmonolaurat und Sorbitanmonolaurat + 20 EO; Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 0 bis 30 Ethylenoxid- und/oder Propylenoxid-Einheiten, wie beispielsweise Nonylphenol + 4 EO, Nonylphenol + 9 EO, Octylphenol + 3 EO und Octylphenol + 8 EO, sowie Alkylpolyglycoside. Als nichtionische Tenside eignen sich insbesondere Alkylmono- und -oligoglycoside und deren ethoxylierte Analoga. Bevorzugt sind solche Alkylpolyglykoside der Formel RO-(Z)ₓ, bei denen R im Wesentlichen aus C₈-C₁₈-Alkylgruppen besteht. Als Zuckerbaustein Z können beliebige Mono- oder Oligosaccharide, wie Glucose, Fructose, Galactose, Arabinose und Sucrose, eingesetzt werden. Glucose ist besonders bevorzugt. Die erfindungsgemäß verwendbaren Alkylpolyglykoside enthalten im Schnitt 1,1 bis 5 Zuckereinheiten. Die zusätzlichen nichtionischen Tenside werden in bevorzugt in einer Gesamtmenge von 0,1 bis 45 Gew.%, bevorzugt 1 bis 30 Gew.% und ganz besonders bevorzugt von 1 bis 20 Gew.%, bezogen auf die Gesamtmenge des anwendungsbereiten Mittels, eingesetzt.

Erfindungsgemäß bevorzugt sind kationische Tenside vom Typ der quartären Ammoniumverbindungen, der Esterquats und der Amidoamine. Bevorzugte quaternäre Ammoniumverbindungen sind Ammoniumhalogenide, wie Alkyltrimethylammoniumchloride, Dialkyldimethylammoniu chloride und Trialkylmethylammoniumchloride sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Ebenfalls erfindungsgemäß vorteilhaft sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen (Warenzeichen Stepantex, Dehyquart und Armocare). Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, bevorzugt Dimethicon, Farbstoffe zum Anfärben des Mittels, Antischuppenwirkstoffe, Lichtschutzmittel, Proteine und Proteinhydrolysate, die gegebenenfalls geeignet modifiziert sind, Wirkstoffe wie Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und - distearat sowie PEG-3-distearat, Pigmente, Treibmittel wie Propan-Butan-Gemische, N₂O Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbemittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Die erfindungsgemäßen Färbemittel besitzen durch ihre spezifische Trägerbasis signifikante Vorteile gegenüber dem Stand der Technik im Hinblick auf ihre rheologischen Eigenschaften. Insbesondere zeichnen sich die erfindungsgemäßen Mittel durch eine hohe Lagerstabilität aus. Die Mittel behalten eine stabile Viskosität ohne wesentlichen Anstieg und insbesondere ohne Abfall in der Viskosität, was zu erheblichen Lager- und Anwendungsproblemen führen könnte. Diese Stabilität in der Viskosität gilt insbesondere für die Zubereitung, enthaltend die Oxidationsfarbstoffvorprodukte, aber auch für das anwendungsbereite Mittel nach Vermischen von Oxidationsmittelzubereitung und Zubereitung, enthaltend die Oxidationsfarbstoffvorprodukte.

Erfindungsgemäß bevorzugte Mittel besitzen eine Viskosität von 8000 bis 35000 mPa·s, insbesondere von 12000 bis 25000 mPa·s, jeweils gemessen nach Brookefield mit Spindel No. 5 bei 20-23°C und einer Rotationsgeschwindigkeit von 4 U·min⁻¹.

Weiterhin besitzen die Mittel gegenüber herkömmlichen Mitteln Vorteile hinsichtlich ihrer Scherempfindlichkeit. Eine stärkere Scherung führt dabei zu einer leichten Viskositätserhöhung gegenüber herkömmlichen Mitteln, bei denen gelegentlich sogar eine unerwünschte Viskositätserniedrigung festgestellt wird, wodurch es zu Problemen bei Abfüllung und Lagerung kommen kann.

Ein zweiter Gegenstand der vorliegenden Erfindung ist eine Mehrkomponentenverpackungseinheit (Kit-of-Parts), bestehend aus mindestens zwei getrennt voneinander konfektionierten Containern, wobei ein Container (I) ein Färbemittel (A), enthaltend in einem kosmetischen Träger mindestens ein Oxidationsfarbstoffvorprodukt, und ein weiterer Container (II) eine wässrige Oxidationsmittelzubereitung (B), enthaltend mindestens ein Oxidationsmittel, enthält, dadurch gekennzeichnet, dass das Färbemittel (A)
mindestens eine nichtionische Emulgatorkombination aus mindestens zwei ethoxylierten, linearen Fettalkoholen jeweils mit 8 bis 22 Kohlenstoffatomen, wobei mindestens einer der ethoxylierten Fettalkohole einen mittleren, durchschnittlichen Ethoxylierungsgrad von 15 bis 35 Mol Ethylenoxid besitzt und mindestens einer der ethoxylierten Fettalkohole einen hohen, durchschnittlichen Ethoxylierungsgrad von 40 bis 100 Mol Ethylenoxid besitzt, und mindestens eine Kombination grenzflächenaktiver Substanzen, enthaltend mindestens ein anionisches Tensid, mindestens ein zwitterionisches Tensid und mindestens ein amphoteres Tensid,
enthält und dadurch gekennzeichnet, dass die Kombination grenzflächenaktiver Substanzen ein Gewichtsverhältnis zwischen der Gesamtmenge an anionischen Tensiden und der Gesamtmenge an amphoteren und zwitterionischen Tensiden besitzt, dessen Wert zwischen 3 und 0,5 liegt.

Eine Ausführungsform dieses Erfindungsgegenstands ist dadurch gekennzeichnet, dass das Gewichtsverhältnis in der Kombination grenzflächenaktiver Substanzen zwischen der Gesamtmenge an anionischen Tensiden und der Gesamtmenge an amphoteren und zwitter-ionischen Tensiden einen Wert zwischen 3 und 0,3 besitzt.

Wird eine besonders starke Aufhellwirkung durch Einsatz von Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen. Unter Container wird im Rahmen der vorliegenden Erfindung eine Umhüllung verstanden, die in Form einer gegebenenfalls wieder-verschließbaren Flasche, einer Dose, eines Tütchens, eines Sachets oder ähnlichen Umhüllungen vorliegt. Dabei kann die Umhüllung aus Kunststoff, Glas, (Metall-) blech, Karton, Papier oder einem Verbundstoff gefertigt sein.

Weiterhin enthält die Mehrkomponentenverpackungseinheit (Kit-of-Parts) zusätzlich eine Gebrauchsleitung. Darüber hinaus kann es bevorzugt sein, wenn weiterhin eine Applikationshilfe, wie beispielsweise ein Kamm oder ein Pinsel oder eine Anmischschale, und/oder eine persönliche Schutzausrüstung, wie beispielsweise Einweg-Handschuhe dem Kit beigefügt ist.

Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der vorliegenden Erfindung ist ein Verfahren zum Färben menschlicher Haare, bei dem ein Mittel des ersten Erfindungsgegenstandes auf das Haar aufgetragen wird, für eine Einwirkzeit von 2 bis 30 Minuten, bevorzugt von 5 bis 15 Minuten auf dem Haar belassen wird, und anschließend das Haar ausgespült wird.

Ein weiterer Gegenstand der vorliegenden Erfindung ist weiterhin ein Verfahren zum Färben menschlicher Haare, bei dem ein Mittel unmittelbar vor der Anwendung durch Vermischung der Komponenten der Mehrkomponentenverpackungseinheit des zweiten Erfindungsgegenstands hergestellt wird, das nunmehr anwendungsbereite Mittel auf das Haar aufgetragen wird, für eine Einwirkzeit von 2 bis 30 Minuten, bevorzugt von 5 bis 15 Minuten, auf dem Haar belassen wird, und anschließend das Haar ausgespült wird.

Die im Vergleich zu Handelsprodukten kurze Einwirkzeit in den erfindungsgemäßen Verfahren garantiert bei gleichbleibenden Färbeergebnis ebenfalls eine verringerte Schädigung der Haare durch die verkürzte Kontaktzeit von schädigenden Substanzen mit dem Haar.

Die Anwendungstemperaturen beim erfindungsgemäßen Färbeverfahren können in einem Bereich zwischen 15 und 45 °C liegen. Nach der Einwirkungszeit wird das Haarfärbemittel durch Ausspülen, gegebenenfalls mit Hilfe eines Shampoos, von dem zu färbenden Haar entfernt. Das Nachwaschen mit einem Shampoo kann entfallen, wenn ein stark tensidhaltiger Träger, z. B. ein Färbeshampoo, verwendet wurde. Bezüglich weiterer bevorzugter Ausführungsformen der erfindungsgemäßen Verfahren gilt mutatis mutandis das zu den erfindungsgemäßen Mitteln Gesagte.

Schließlich hat sich gezeigt, dass sich die rheologischen Eigenschaften, insbesondere Viskositätsstabilität und Scherempfindlichkeit, von Färbemitteln durch Verwendung der erfindungsgemäßen Mittel deutlich verbessern lassen.

Ein weiterer Gegenstand der vorliegenden Erfindung ist daher Verwendung eines Mittels des ersten Erfindungsgegenstands zur Verbesserung der rheologischen Eigenschaften, insbesondere der Viskositätsstabilität und der Scherempfindlichkeit.

Die nachfolgenden Beispiele sollen bevorzugte Ausführungsformen der Erfindung erläutern, ohne sie jedoch einzuschränken.

### Beispiele

Herstellung von Färbecremes (Mengengaben jeweils als prozentuale Gewichtsanteile)

| Rohstoff | E1 | E2 |
|---|---|---|
| Lanette D | 12,0 | 12,0 |
| Lorol techn. | 6,0 | 6,0 |
| Eumulgin B2 | 0,5 | 0,5 |
| Eumulgin B3 | 0,5 | 0,5 |
| Mergital CS 50 A | 1,4 | 1,4 |
| Texapon NSO | 3,0 | 3,0 |
| Dehyton K | 2,0 | 2,0 |
| Amphoterge K-2 | 2,0 | 2,0 |
| 1,2-Propandiol | 1,0 | 1,0 |
| Xanthan | 0,2 | 0,2 |
| Merquat 281 | 1,5 | 1,5 |
| Ammoniumsulfat, techn. rein | 2,0 | 2,0 |
| Natriumsulfit, wasserfrei, 96% | 0,2 | 0,2 |
| Ascorbinsäure | 0,2 | 0,2 |
| L-Arginin | 1,0 | 1,0 |
| p-Toluylendiaminsulfat | 3,50 | 1,44 |
| Resorcin | 1,25 | 0,27 |
| 4-Chlorresorcin | -- | 0,14 |
| 2-Methylresorcin | 0,23 | 0,32 |
| 3-Aminophenol | 0,19 | 0,12 |
| 2-Amino-3-hydroxypyridin | 0,11 | -- |
| HEDP, 60% | 0,2 | 0,2 |
| Natriumsilikat 40 / 42 | 0,5 | 0,5 |
| Monoethanolamin | 5,5 | 5,5 |
| Parfum | qs | qs |
| Wasser | ad 100 | ad 100 |

| | | |
|---|---|---|
| Lanette^{®} D C₁₆-C₁₈-Fettalkohol (INCI-Bezeichnung: Cetearyl alcohol) (Cognis) Lorol^{®} tech. C₁₂-C₁₈-Fettalkohol (INCI-Bezeichnung: Coconut alcohol) (Cognis) Eumulgin^{®} B2 C₁₆-C₁₈-Fettalkohol, ethoxyliert (20 EO) (INCI-Bezeichnung: Ceteareth-20) (Cognis) Eumulgin^{®} B3 C₁₆-C₁₈-Fettalkohol, ethoxyliert (30 EO) (INCI-Bezeichnung: Ceteareth-20) (Cognis) Mergital^{®} CS 50 C₁₆-C₁₈-Fettalkohol, ethoxyliert (50 EO) (INCI-Bezeichnung: Ceteareth-50) (Cognis) Texapon^{®} NSO C₁₂-Fettalkoholsulfat, ethoxyliert (2 EO) Natrium-Salz (27% Aktivsubstanz; INCI-Bezeichnung: Sodium Laureth Sulfate) (Cognis) Dehyton^{®}K N,N-Dimethyl-N-(cocosalkylamidopropyl)ammonium acetobetain (30% Aktivsubstanz; INCI-Bezeichnung: Cocamidopropyl Betaine) (Cognis) Amphoterge^{®} K-2 (40% Aktivsubstanz; INCI-Bezeichnung: Disodium Cocoamphodipropionate) (Lonza) Merquat^{®} 281 Dimethyldiallylammoniumchlorid/Acrylsäure Copolymer (40% Aktivsubstanz; INCI-Bezeichnung: Polyquaternium-22) (Nalco) Natriumsilikat 40/42 Natronwasserglas | | |

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

Die erfindungsgemäßen Mittel enthalten dabei:
a) eine Kombination grenzflächenaktiver Substanzen, bei der das Gewichtsverhältnis zwischen der Gesamtmenge an anionischen Tensiden (Texapon NSO; 0,81 % Aktivsubstanz) und der Gesamtmenge an amphoteren und zwitterionischen Tensiden (Dehyton K; 0,60 % AS und Amphoterge K-2; 0,80 % AS) einen Wert von [0,81/(0,60+0,80)] = 0,58 besitzt;
   und
b) eine nichtionische Emulgatorkombination, bei der das Gewichtsverhältnis zwischen ethoxylierten Fettalkoholen mit einem mittleren, durchschnittlichen Ethoxylierungsgrad (Eumulgin B2 und Eumulgin B3; je 0,5 % AS) und ethoxylierten Fettalkoholen mit einem hohen, durchschnittlichen Ethoxylierungsgrad (Mergital CS 50, 1,4 % AS) einen Wert von [(0,5+0,5)/1,4] = 0,71 besitzt.

Es wurden Färbecremes E1 und E2 mit hoher Viskositätsstabilität, insbesondere während der Lagerung (3 Monate), erhalten.

Mittel, bei denen eine der grenzflächenaktiven Verbindungen Texapon NSO, Dehyton K oder Amphoterge K-2 nicht eingearbeitet worden war, wiesen deutlich schlechtere Viskositätsstabilitäten während der Lagerung auf, so dass deren Anwendbarkeit eingeschränkt war.

## Patentansprüche

1. Mittel zum oxidativen Färben keratinischer Fasern, insbesondere menschlicher Haare, enthaltend in einem kosmetischen Träger mindestens ein Oxidationsfarbstoffvorprodukt sowie
a) mindestens eine nichtionischen Emulgatorkombination aus mindestens zwei ethoxylierten, linearen Fettalkoholen mit jeweils 8 bis 22 Kohlenstoff-Atomen, wobei mindestens einer der ethoxylierten Fettalkohole einen mittleren, durchschnittlichen Ethoxylierungsgrad von 15 bis 35 Mol Ethylenoxid besitzt und mindestens einer der ethoxylierten Fettalkohole einen hohen, durchschnittlichen Ethoxylierungsgrad von 40 bis 100 Mol Ethylenoxid besitzt, und
b) mindestens eine Kombination grenzflächenaktiver Substanzen, enthaltend mindestens ein anionisches Tensid, mindestens ein zwitterionisches Tensid und mindestens ein amphoteres Tensid,
**dadurch gekennzeichnet, dass** die Kombination grenzflächenaktiver Substanzen ein Gewichtsverhältnis zwischen der Gesamtmenge an anionischen Tensiden und der Gesamtmenge an amphoteren und zwitterionischen Tensiden besitzt, dessen Wert zwischen 3 und 0,5 liegt.

2. Mittel nach Anspruch 1, **dadurch gekennzeichnet, dass** die Kombination grenzflächenaktiver Substanzen ein Gewichtsverhältnis zwischen der Gesamtmenge an anionischen Tensiden und der Gesamtmenge an amphoteren und zwitterionischen Tensiden besitzt, dessen Wert zwischen 2 und 0,5, liegt.

3. Mittel nach einem der Ansprüche 1 bis 2, **dadurch gekennzeichnet, dass** der ethoxylierte, lineare Fettalkohol mit einen hohen, durchschnittlichen Ethoxylierungsgrad des anwendungsbereiten Mittels einen durchschnittlichen Ethoxylierungsgrad von 40 bis 60, bevorzugt 45 bis 55 besitzt.

4. Mittel nach einem der Ansprüche 1 bis 3, **dadurch gekennzeichnet, dass** das Gewichtsverhältnis der Menge der ethoxylierten Fettalkohole mit einem mittleren, durchschnittlichen Ethoxylierungsgrad zu der Menge der ethoxylierten Fettalkohole mit einem hohen, durchschnittlichen Ethoxylierungsgrad im anwendungsbereiten Mittel einen Wert von 3:1 bis 1:5, bevorzugt von 2:1 bis 1:3, besitzt.

5. Mittel nach einem der Ansprüche 1 bis 4, **dadurch gekennzeichnet, dass** das Mittel zusätzlich mindestens einen polymeren Verdicker, bevorzugt in einer Menge von 0,005 bis 1,0 Gew.-%, bezogen auf das Gesamtgewicht der Färbemittels, enthält.

6. Mittel nach Anspruch 5, **dadurch gekennzeichnet, dass** der polymere Verdicker des Mittels ein verdickendes, polysaccharidisches Polymer, bevorzugt Xanthan, ist.

7. Mittel nach einem der Ansprüche 1 bis 6, **dadurch gekennzeichnet, dass** das Mittel eine Kombination von mindestens zwei Alkalisierungsmitteln enthält, wobei mindestens ein Alkalisierungsmittel Ammoniak oder ein Alkanolamin ist, ausgewählt aus Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol, 2-Aminobutanol, N,N-Dimethyl-ethanolamin, Methylglucamin, Triethanolamin, Diethanolamin und Triisopropanolamin, und wobei mindestens ein Alkalisierungsmittel eine basische Aminosäure ist.

8. Mittel nach Anspruch 7, **dadurch gekennzeichnet, dass** das Alkanolamin Monoethanolamin ist.

9. Mittel nach einem der Ansprüche 7 bis 8, **dadurch gekennzeichnet, dass** die basische Aminosäure ausgewählt ist aus der Gruppe, die gebildet wird aus Lysin, Guanidin, Ornithin und Arginin.

10. Mittel nach einem der Ansprüche 1 bis 9, **dadurch gekennzeichnet, dass** das Mittel zusätzlich mindestens einen direktziehenden Farbstoff enthält.

11. Mehrkomponentenverpackungseinheit (Kit-of-Parts), bestehend aus mindestens zwei getrennt voneinander konfektionierten Containern, wobei ein Container (I) ein Färbemittel (A), enthaltend in einem kosmetischen Träger mindestens ein Oxidationsfarbstoffvorprodukt, und ein weiterer Container (II) eine wässrige Oxidationsmittelzubereitung (B), enthaltend mindestens ein Oxidationsmittel, enthält, **dadurch gekennzeichnet, dass** das Färbemittel (A) mindestens eine nichtionischen Emulgatorkombination aus mindestens zwei ethoxylierten, linearen Fettalkoholen mit jeweils 8 bis 22 Kohlenstoffatomen, wobei mindestens einer der ethoxylierten Fettalkohole einen mittleren, durchschnittlichen Ethoxylierungsgrad von 15 bis 35 Mol Ethylenoxid besitzt und mindestens einer der ethoxylierten Fettalkohole einen hohen, durchschnittlichen Ethoxylierungsgrad von 40 bis 100 Mol Ethylenoxid besitzt, und mindestens eine Kombination grenzflächenaktiver Substanzen, enthaltend mindestens ein anionisches Tensid, mindestens ein zwitterionisches Tensid und mindestens ein amphoteres Tensid, enthält, **dadurch gekennzeichnet, dass** das Gewichtsverhältnis in der Kombination grenzflächenaktiver Substanzen zwischen der Gesamtmenge an anionischen Tensiden und der Gesamtmenge an amphoteren und zwitter-ionischen Tensiden einen Wert zwischen 3 und 0,5 besitzt.

12. Verfahren zum Färben menschlicher Haare, bei dem ein Mittel unmittelbar vor der Anwendung durch Vermischung der Komponenten der Mehrkomponentenverpackungseinheit nach Anspruch 11 hergestellt wird, das nunmehr anwendungsbereite Mittel auf das Haar aufgetragen wird, für eine Einwirkzeit von 2 bis 30 Minuten, bevorzugt von 5 bis 15 Minuten, auf dem Haar belassen wird, und anschließend das Haar ausgespült wird.

13. Verwendung eines Mittels gemäß einem der Ansprüche 1 bis 10 zur Verbesserung der rheologischen Eigenschaften, insbesondere der Viskositätsstabilität und der Scherempfindlichkeit.

## Claims

1. An agent for oxidatively dyeing keratinous fibers, in particular human hair, containing in a cosmetic carrier at least one oxidation dye precursor and
a) at least one non-ionic emulsifier combination consisting of at least two ethoxylated, linear fatty alcohols each having 8 to 22 carbon atoms, at least one of the ethoxylated fatty alcohols having a mean average degree of ethoxylation of from 15 to 35 mol ethylene oxide and at least one of the ethoxylated fatty alcohols having a high average degree of ethoxylation of from 40 to 100 mol ethylene oxide, and
b) at least one combination of surface-active substances, containing at least one anionic surfactant, at least one zwitterionic surfactant and at least one amphoteric surfactant,
**characterized in that** the combination of surface-active substances has a weight ratio between the total amount of anionic surfactants and the total amount of amphoteric and zwitterionic surfactants, the value of which is between 3 and 0.5.

2. The agent according to claim 1, **characterized in that** the combination of surface-active substances has a weight ratio between the total amount of anionic surfactants and the total amount of amphoteric and zwitterionic surfactants, the value of which is between 2 and 0.5.

3. The agent according to either claim 1 or claim 2, **characterized in that** the ethoxylated, linear fatty alcohol having a high average degree of ethoxylation of the ready-to-apply agent has an average degree of ethoxylation of from 40 to 60, preferably 45 to 55.

4. The agent according to one of claims 1 to 3, **characterized in that** the weight ratio of the amount of the ethoxylated fatty alcohols having a mean average degree of ethoxylation to the amount of the ethoxylated fatty alcohols having a high average degree of ethoxylation in the ready-to-apply agent has a value of from 3:1 to 1:5, preferably from 2:1 to 1:3.

5. The agent according to one of claims 1 to 4, **characterized in that** the agent additionally contains at least one polymer thickener, preferably in an amount of 0.005 to 1.0 wt.%, based on the total weight of the dyeing agent.

6. The agent according to claim 5, **characterized in that** the polymer thickener of the agent is a thickening, polysaccharide polymer, preferably xanthan.

7. The agent according to one of claims 1 to 6, **characterized in that** the agent contains a combination of at least two alkalizing agents, at least one alkalizing agent being ammonia or an alkanolamine, selected from monoethanolamine, monoisopropanolamine, 2-amino-2-methylpropanol, 2-amino-2-methyl-1,3-propanediol, 2-amino-2-ethyl-1,3-propanediol, 2-amino-2-methybutanol, 2-aminobutanol, N,N-dimethyl-ethanolamine, methylglucamine, triethanolamine, diethanolamine and triisopropanolamine, and at least one alkalizing agent being a basic amino acid.

8. The agent according to claim 7, **characterized in that** the alkanolamine is monoethanolamine.

9. The agent according to one of claims 7 to 8, **characterized in that** the basic amino acid is selected from the group formed of lysine, guanidine, ornithine and arginine.

10. The agent according to one of claims 1 to 9, **characterized in that** the agent additionally contains at least one direct dye.

11. A kit of parts, consisting of at least two separately packaged containers, a container (I) containing a dyeing agent (A), which contains in a cosmetic carrier at least one oxidation dye precursor, and another container (II) containing an aqueous oxidizing agent preparation (B), which contains at least one oxidizing agent, **characterized in that** the dyeing agent (A) contains at least one non-ionic emulsifier combination composed of at least two ethoxylated linear fatty alcohols each having 8 to 22 carbon atoms, at least one of the ethoxylated fatty alcohols having a mean average degree of ethoxylation of from 15 to 35 mol ethylene oxide and at least one of the ethoxylated fatty alcohols having a high average degree of ethoxylation of from 40 to 100 mol ethylene oxide, and at least one combination of surface-active substances, containing at least one anionic surfactant, at least one zwitterionic surfactant and at least one amphoteric surfactant, **characterized in that** the weight ratio in the combination of surface-active substances between the total amount of anionic surfactants and the total amount of amphoteric and zwitterionic surfactants has a value of between 3 and 0.5.

12. A method for coloring human hair, wherein an agent is produced directly before application by mixing the components of the kit of parts according to claim 11, the now ready-to-apply agent is applied to the hair, left on the hair for an application time of 2 to 30 minutes, preferably 5 to 15 minutes, and then rinsed off the hair.

13. The use of an agent according to one of claims 1 to 10 for improving the rheological properties, in particular the viscosity stability and the shear sensitivity.

## Revendications

1. Produit de coloration oxydative de fibres de kératine, en particulier de cheveux humains, contenant dans un vecteur cosmétique au moins un précurseur de coloration oxydative ainsi que :
a) au moins une combinaison d'émulsifiants non-ioniques d'au moins deux alcools gras linéaires éthoxylés avec respectivement 8 à 22 atomes de carbone, au moins un des alcools gras éthoxylés possédant un degré d'éthoxylation moyen médium de 15 à 35 M d'oxyde d'éthylène, et au moins un des alcools gras éthoxylés possédant un degré d'éthoxylation moyen élevé de 40 à 100 M d'oxyde d'éthylène, et
b) au moins une combinaison de tensioactifs contenant au moins un tensioactif anionique, au moins un tensioactif zwitterionique et au moins un tensioactif amphotère,
**caractérisé en ce que** la combinaison de tensioactifs possède un rapport pondéral entre la quantité totale de tensioactifs anioniques et la quantité totale de tensioactifs zwitterioniques et amphotères dont la valeur se situe entre 3 et 0,5.

2. Produit selon la revendication 1, **caractérisé en ce que** la combinaison de tensioactifs possède un rapport pondéral entre la quantité totale de tensioactifs anioniques et la quantité totale de tensioactifs zwitterioniques et amphotères dont la valeur se situe entre 2 et 0,5.

3. Produit selon la revendication 1 ou 2, **caractérisé en ce que** l'alcool gras linéaire éthoxylé de degré d'éthoxylation moyen élevé du produit prêt à l'emploi possède un degré d'éthoxylation moyen entre 40 et 60, préférentiellement entre 45 et 55.

4. Produit selon une des revendications 1 à 3, **caractérisé en ce que** le rapport pondéral de la quantité d'alcool gras éthoxylé de degré d'éthoxylation moyen médium à la quantité d'alcool gras éthoxylé de degré d'éthoxylation moyen élevé dans le produit prêt à l'emploi a une valeur entre 3:1 et 1:5, préférentiellement entre 2:1 et 1:3.

5. Produit selon une des revendications 1 à 4, **caractérisé en ce que** le produit contient en plus un épaississant polymère, préférentiellement à hauteur de 0,005 à 1,0 % en poids rapporté au poids total du colorant.

6. Produit selon la revendication 5, **caractérisé en ce que** l'épaississant polymère du produit est un polymère épaississant polysaccharide, préférentiellement du xanthane.

7. Produit selon une des revendications 1 à 6, **caractérisé en ce que** le produit contient une combinaison d'au moins deux alcalinisants, au moins un alcalinisant étant de l'ammoniac ou une alcanolamine choisi parmi la monoéthanolamine, la monoisopropanolamine, le 2-amino-2-méthylpropanol, le 2-amino-2-méthyl-1,3-propanediol, le 2-amino-2-éthyl-1,3-propanediol, le 2-amino-2-méthylbutanol, le 2-aminobutanol, la N,N-diméthyléthanolamine, la méthylglucosamine, la triéthanolamine, la diéthanolamine et la triisopropanolamine, et
au moins un alcalinisant étant un acide aminé basique.

8. Produit selon la revendication 7, **caractérisé en ce que** l'alcanolamine est de la monoéthanolamine.

9. Produit selon la revendication 7 ou 8, **caractérisé en ce que** l'acide aminé basique est choisi dans le groupe constitué de la lysine, de la guanidine, de l'ornithine et de l'arginine.

10. Produit selon une des revendications 1 à 9, **caractérisé en ce que** le produit contient en plus au moins un colorant direct.

11. Kit à emballage multicomposant (kit of parts) constitué d'au moins deux récipients séparés l'un de l'autre, un récipient (I) contenant au moins un précurseur de colorant d'oxydation dans un vecteur cosmétique contenant un colorant (A), et un autre récipient (II) contenant au moins un oxydant contenant une préparation aqueuse d'oxydant (B), **caractérisé en ce que** le colorant (A) contient :
au moins une combinaison d'émulsifiants non-ioniques constitué d'au moins deux alcools gras linéaires éthoxylés avec respectivement 8 à 22 atomes de carbone, au moins un des alcools gras éthoxylés possédant un degré d'éthoxylation moyen médium de 15 à 35 M d'oxyde d'éthylène, et au moins un des alcools gras éthoxylés possédant un degré d'éthoxylation moyen élevé de 40 à 100 M d'oxyde d'éthylène, et
au moins une combinaison de tensioactifs contenant au moins un tensioactif anionique, au moins un tensioactif zwitterionique et au moins un tensioactif amphotère,
**caractérisé en ce que** le rapport pondéral dans la combinaison de tensioactifs entre la quantité totale de tensioactifs anioniques et la quantité totale de tensioactifs zwitterioniques et amphotères dont la valeur se situe entre 3 et 0,5.

12. Procédé de coloration de cheveux humains, dans lequel on fabrique un produit immédiatement avant utilisation par mélange des composants du kit à emballage multicomposant selon la revendication 11, on applique le produit prêt à l'emploi sur les cheveux, on le laisse agir sur les cheveux de 2 à 30 minutes, de préférence de 5 à 15 minutes, puis on rince les cheveux.

13. Utilisation d'un produit selon une des revendications 1 à 10 pour améliorer les propriétés rhéologiques, en particulier la stabilité de la viscosité et la sensibilité au cisaillement.
